# EUROPEAN PATENT APPLICATION

(11) **EP 4 786 526 A1**
(43) Date of publication of application: **05.08.2026**
(21) Application number: 24871779.5
(22) Date of filing: 05.09.2024
(51) Int. Cl.: C08G 81/00, A61K 9/10, A61K 9/51, A61K 31/765, A61K 41/00, A61N 5/10, A61P 35/00, A61P 43/00

(54) **HYDROPHILIC-HYDROPHOBIC COPOLYMERS CARRYING DICHLOROACETIC ACID ON SIDE CHAIN AND MEDICAL USE THEREOF**

(30) Priority: 28.09.2023 JP 2023168768
(71) Applicant: University of Tsukuba, Ibaraki 305-8577 (JP); National University Corporation Hokkaido University, Hokkaido 060-0808 (JP)
(72) Inventor: NAGASAKI, Yukio, Tsukuba-shi, Ibaraki 305-8577 (JP); YASUI, Hironobu, Sapporo-shi, Hokkaido 060-0808 (JP); YAMADA, Sato, Sapporo-shi, Hokkaido 060-0808 (JP); INANAMI, Osamu, Sapporo-shi, Hokkaido 060-0808 (JP)
(74) Representative: Kraus & Lederer PartGmbB
(86) International application number: PCT/JP2024/031887
(87) International publication number: WO 2025/069980

(57) **Abstract**

The present invention discloses copolymers which are represented by general formulae (I), (II-a), and (II-b) (The variable groups or moieties in the formulae are defined in the specification. In particular, R₁ or R₂ includes a dichloroacetyl group). These copolymers or polymeric micelles which are formed from these copolymers in an aqueous medium have a tumor-specific radiation enhancement effect and enhance the therapeutic effect of radiation therapy on tumors. Consequently, the present invention provides a medical use of these copolymers or polymeric micelles for exerting such an effect.

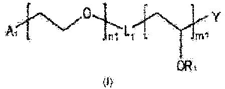

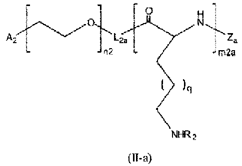

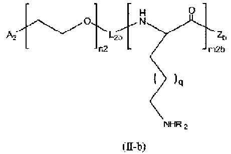

## Description

### Technical Field

The present invention relates to a hydrophilic-hydrophobic copolymer having a block having dichloroacetic acid in a hydrophobic segment side chain via an ester or amide bond and poly(ethylene glycol) as a block of a hydrophilic segment, self-assembled nanoparticles (or polymeric micelles) of the copolymer, and medical use of the copolymer or nanoparticles, more specifically, use as a pharmaceutical preparation for enhancing a therapeutic effect by combination use in a tumor-specific radiosensitizer and radiation therapy for tumors.

### Background Art

From the viewpoint that cancer cells exhibited an increased dependence on glycolysis for ATP production even in the presence of oxygen (Warburg effect) and the like, application of a glycolytic inhibitor in cancer therapy has attracted attention. Since dichloroacetic acid (DCA) is an inhibitor of pyruvate dehydrogenase kinase (PDK) and is a type of the glycolytic inhibitor, clinical trials have been conducted; however, DCA has not been approved as an anticancer therapeutic agent. This is because the DCA may show an antitumor effect smaller than expected in vitro and in vivo. Thus, in cancer treatment, combination use of the DCA and a certain type of chemotherapeutic agent and combination use of radiation therapy have been attempted. These studies have demonstrated the radiosensitizing effect of the DCA on certain tumor cells (see F Zwicker et al., Strahlenther Onkol. 2013; 189 (8): 684-692 (hereinafter referred to as Non-Patent Document 1)). The DCA has not been approved as the cancer therapeutic agent described above because the DCA having a simple structure has a rapid metabolism and insufficient accumulation in solid tumors, and thus, although the DCA exhibits an effect in a cell test, the DCA cannot exhibit a sufficient effect in an animal experiment, and further, when the dose is increased, toxicity of the DCA itself has some impact, and the like.

As a measure to solve such issues, if it can be effectively applied to the DCA, according to the first report by Maeda (see Hiroshi Maeda, Cancer Science, 2013; 104 (7): 779-789 (hereinafter referred to as Non-Patent Document 2)), one candidate is to modify the DCA so as to be covalently bonded to a certain polymer compound to be polymerized, and thus to exhibit a blood vessel permeability/retention enhancement effect (EPR effect).

On the other hand, some of the present inventors have hitherto shown that by introducing a chain transfer group and the like into a poly(ethylene glycol) terminal and radically polymerizing a short-chain fatty acid vinyl, a short-chain fatty acid such as propionic acid or butyric acid is introduced into a hydrophobic segment side chain by an ester bond, and core-shell type nanoparticles formed by them function as therapeutic agents for diabetes mellitus, nonalcoholic steatohepatitis (NASH), and hepatic fibrosis, or an anticancer agent (see WO 2021/024906 A1 (hereinafter referred to as Patent Document 1)).

However, unlike a short-chain fatty acid ester such as esters of propionic acid and butyric acid, a dichloroacetic acid ester has high hydrolyzability and chain mobility, and it has been difficult to synthesize a target polymer compound even if the DCA is attempted to be polymerized by the same technique as the technique described in Non-Patent Document 2 or Patent Document 1.

### Summary of Invention

### Technical Problem

Therefore, an object of the present invention is to provide an invention in which dichloroacetic acid (DCA) is effectively polymerized, and at the same time, a polymer compound thus obtained or any structure formed from the polymer compound has characteristics of solving or correcting issues such as rapid metabolism and insufficient accumulation in solid tumors, which are disadvantages associated with use of DCA itself.

### Solution to Problem

The present inventors have found that when PEG-b-poly (short-chain fatty acid vinyl) produced according to the description of Patent Document 1 is hydrolyzed and then replaced with a dichloroacetic acid ester instead of introducing a chain transfer group and the like into a poly(ethylene glycol) (PEG) terminal and directly radically polymerizing a short-chain fatty acid vinyl, a target polymer compound can be effectively produced, and nano-sized polymeric micelles or nanoparticles formed from the polymer compound thus produced through self-assembly in an aqueous medium (water, aqueous solution of water-miscible organic solvent, and the like) overcome the disadvantages associated with the use of DCA itself described above, and at least exhibit tumor-specific radiosensitivity. As another method, the present inventors have also found that a target polymer compound can be effectively produced by introducing a dichloroacetyl group by reacting dichloroacetic anhydride with a side chain amino group (each δ- or ε- position) of PEG-b-poly (ornithine) or PEG-b-poly (lysine) provided in advance. In addition, it has been found that the polymeric micelles or nanoparticles formed from the polymer compound thus produced also have properties substantially equivalent to those of the PEG-b-poly (short-chain fatty acid vinyl) -derived copolymer.

Accordingly, the main subject matter or features disclosed herein may include the following.

### Subject matter 1:

A hydrophilic-hydrophobic copolymer comprising: a block comprising a poly(ethylene glycol) segment; and a block comprising a polymer segment carrying dichloroacetic acid in a side chain,
wherein the hydrophilic-hydrophobic copolymer is represented by Formula (I), (II-a), or (II-b). 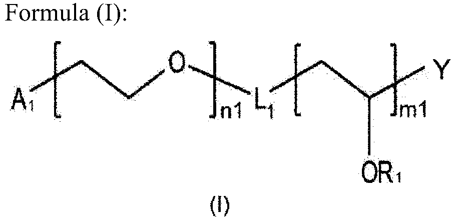
where
A₁ represents
   (i) unsubstituted or substituted C₁-C₁₂ alkyloxy, a substituent in a case of being substituted is a formyl or a group of Formula R^{a}R^{b}CH-,(where R^{a} and R^{b} are independently C₁-C₄ alkyloxy or R^{a} and R^{b} together represent -OCH₂CH₂O-, -O(CH₂)₃O-, or -O(CH₂)₄O-), or
   (ii) a group represented by the following formula: 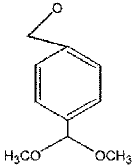
L₁ is selected from groups represented by 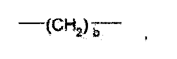 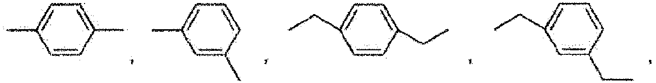
or the group consisting of (CH₂)_{c}S, CO(CH₂)_{c}S, (CH₂)_{c}NH, (CH₂)_{c}CO, CO, OCOO, and CONH, with the proviso that b is an integer of from 2 to 6, and c is each independently an integer of from 1 to 5,
R₁ is a hydrogen atom, a C₁-C₆ alkylcarbonyl, or C(=O)CHCl₂, and each R₁ is optionally different, with the proviso that 20% or more of m1 is C(=O)CHCl₂,
Y is a hydrogen atom, SH, S(C=S)-Ph, S(=S)OCH₂CH₃, a hydroxyl group, a C₁-C₆ alkyloxy group, or an aryl-C₁-C₂ alkyloxy group,
m1 is an integer of from 5 to 1000, and
n1 is an integer of from 5 to 500. 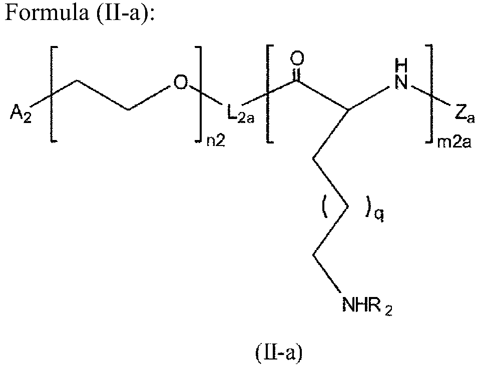
where
A₂ has the same meaning as defined for A₁ of Formula (I)
L₂ₐ represents (CH₂)ₐ-NH or (CH₂)ₐ-O, with the proviso that a is each independently an integer of from 1 to 6,
R₂ is a hydrogen atom, benzyloxycarbonyl (Cbz), t-butoxycarbonyl, C₁-C₆ alkylcarbonyl, or C(=O)CHCl₂, and each R₂ is optionally different, with the proviso that 20% or more of m2a is C(=O)CHCl₂,
Za represents H, C₁-C₂₁ alkylcarbonyl, substituted C₁-C₄ alkylcarbonyl, unsubstituted or substituted C₃-C₇ cycloalkylcarbonyl, unsubstituted or substituted arylcarbonyl, or unsubstituted or substituted 5 or 6-membered heteroarylcarbonyl, where a substituent in a case of being substituted is optionally one or more C₁-C₄ alkyloxy, phenyl, and a fluorine atom,
m2a is an integer of from 5 to 500,
n2a is an integer of from 5 to 500, and
q is an integer of 1 or 2. 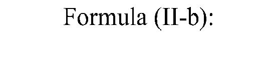 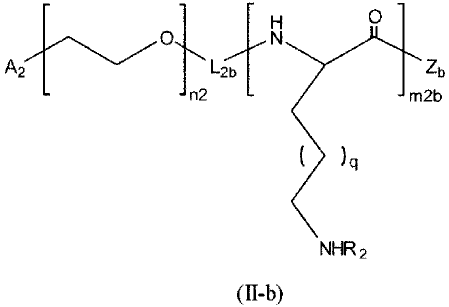
where
A₂ has the same meaning as defined for A₁ of Formula (I)
L_{2b} represents (CH₂)_{b}-C(=O), with the proviso that b is an integer of from 1 to 6,
R₂ is a hydrogen atom, benzyloxycarbonyl (Cbz), t-butoxycarbonyl, C₁-C₆ alkylcarbonyl or C(=O)CHCl₂, and each R₂ is optionally different, with the proviso that 20% or more of m2b is C(=O) CHCl₂,
Zb represents OH, unsubstituted or substituted C₁-C₂₁ alkyloxy, unsubstituted or substituted C₃-C₇ cycloalkyloxy, unsubstituted or substituted aryloxy, or unsubstituted or substituted 5 or 6-membered heteroaryloxy, where a substituent in a case of being substituted is optionally one or more C₁-C₄ alkyloxy, phenyl, and a fluorine atom,
m2b is an integer of from 5 to 500,
n2b is an integer of from 5 to 500, and
q is an integer of 1 or 2.

### Subject matter 2:

The hydrophilic-hydrophobic copolymer according to the subject matter 1, wherein the hydrophilic-hydrophobic copolymer is represented by Formula (I).

### Subject matter 3:

The hydrophilic-hydrophobic copolymer according to the subject matter 1, wherein the hydrophilic-hydrophobic copolymer is represented by Formula (II-a) or (II-b).

### Subject matter 4:

A nano-sized polymeric micelle formed from the hydrophilic-hydrophobic copolymer described in the subject matter 1 in an aqueous medium.

### Subject matter 5:

A tumor-specific radiosensitizer comprising, as an active ingredient, the hydrophilic-hydrophobic copolymer described in the subject matter 1 or a nano-sized polymeric micelle formed from the hydrophilic-hydrophobic copolymer in an aqueous medium.

### Subject matter 6:

A pharmaceutical preparation for enhancing a therapeutic effect by being used in combination with radiation therapy for a tumor, the pharmaceutical preparation comprising, as an active ingredient, the hydrophilic-hydrophobic copolymer described in the subject matter 1 or a nano-sized polymeric micelle formed from the hydrophilic-hydrophobic copolymer in an aqueous medium.

### Subject matter 7:

The hydrophilic-hydrophobic copolymer according to the subject matter 1 or the polymeric micelle according to the subject matter 4 for use as a tumor-specific radiosensitizer.

### Subject matter 8:

The hydrophilic-hydrophobic copolymer according to the subject matter 1 or the nano-sized polymeric micelle according to the subject matter 4 for use in a pharmaceutical preparation for enhancing a therapeutic effect by being used in combination with radiation therapy of a tumor.

### Subject matter 9:

A method for enhancing a therapeutic effect on a tumor, the method comprising administering an effective amount of the hydrophilic-hydrophobic copolymer described in the subject matter 1 or a nano-sized polymeric micelle formed from the hydrophilic-hydrophobic copolymer in an aqueous medium to a patient or an individual who is scheduled to receive or is actually receiving radiation therapy for a tumor.

### Advantageous Effects of Invention

According to the present invention, there are provided: a copolymer represented by Formula (I), (II-a), or (II-b), the administration of which can achieve more dramatic accumulation in tumors than the administration of dichloroacetic acid itself; and a nano-sized polymeric micelle formed therefrom. The polymeric micelles accumulate in the tumor by administration, and as a result, radiation applied to the tumor can be specifically sensitized (tumor-specific radiosensitization). Thus, when the copolymer or nano-sized polymeric micelles derived therefrom are used in combination with radiation therapy for the tumor, the therapeutic effect for the tumor can be enhanced. Since the copolymer and polymeric micelles have low toxicity and are amphiphilic exhibiting hydrophilic-hydrophobicity, the copolymer and polymeric micelles are also useful as carrier materials for encapsulating poorly water-soluble medicines and the like in the polymeric micelle, similarly to the hydrophilic-hydrophobic copolymer known in the art.

### Brief Description of Drawings

FIG. 1 is a ¹H-NMR spectrum of PEG-b-PVA (N1223) in Production Example 1.
FIG. 2 is a size distribution diagram showing a measurement result of dynamic light scattering of a polymeric micelle derived from PEG-b-PVCl₂Ac (N1228) obtained in Production Example 2.
FIG. 3 is a ¹H-NMR spectrum of PEG-b-PVCl₂Ac (N1228) obtained in Production Example 2.
FIG. 4 is a size distribution diagram showing a measurement result of dynamic light scattering of the polymeric micelle derived from PEG-b-POrnOC(=O)CHCl₂ (N1224) obtained in Production Example 3.
FIG. 5 is a ¹H-NMR spectrum of PEG-b-POrnOC(=O)CHCl₂ (N1224) obtained in Production Example 3.
FIG. 6 is a conceptual diagram of an experimental schedule of a colonization method in Test Example 1.
FIG. 7 is a graph display of an influence of EL-Nano^{DCA} and AL-Nano^{DCA} on radiosensitivity and a numerical table summarizing sensitization in Test Example 1.
FIG. 8 is a conceptual diagram of a schedule of an animal test in (a) of Test Example 2.
FIG. 9 is a graphical display of a result of measuring a change in tumor volume associated with the use of DCA alone or in combination with X-ray in (a) of Test Example 2, with a p value after a statistical significance test associated with a graph in a lower part.
FIG. 10 is a graphical display of a result of measuring the change in tumor volume associated with the use of AL-Nano^{DCA} alone or in combination with X-ray in (a) of Test Example 2, with the p value after the statistical significance test associated with a graph in a lower part.
FIG. 11 is a graphical display of a result of measuring the change in tumor volume associated with the use of EL-Nano^{DCA} alone or in combination with X-ray in (a) of Test Example 2, with the p value after the statistical significance test associated with a graph in a lower part.
FIG. 12 is a viability curve of tumor-bearing mice at the time of combined treatment of each DCA, AL-Nano^{DCA}, and EL-Nano^{DCA} with X-ray in (b) of Test Example 2, with the p value after the statistical significant difference test associated with a graph.
FIG. 13 is a conceptual diagram of a schedule of an animal test in (b) of Test Example 2.
FIG. 14 is a graph display of results of measurement of a body weight change of a test animal after single X-ray irradiation, single DCA administration, single AL-Nano^{DCA} administration, and single EL-Nano^{DCA} administration in (b) of Test Example 2.
FIG. 15 is a conceptual diagram of a schedule of the animal test in Test Example 3.
FIG. 16 is a series of photographs of fluorescence microscopic images of fluorescently labeled EL-Nano^{DCA} localized in a tumor after administration of EL-Nano^{DCA} in Test Example 3.
FIG. 17 is a conceptual diagram of a schedule of an experiment using cells in Test Example 4.
FIG. 18 is a graph showing a measurement result of an intracellular ATP amount after EL-Nano^{DCA} administration and X-ray treatment in Test Example 4.

### Detailed Description of the Invention and Best Mode for Carrying Out the Invention

Terms and the like described in connection with the subject matter disclosed herein or the present invention are used as having a meaning or content commonly used in the art unless otherwise specified.

Hereinafter, certain terms and the like disclosed in the present specification can be generally or specifically further described below.

In the present specification, a poly(ethylene glycol) segment and a polymer segment carrying dichloroacetic acid in a side chain are disclosed; however, the term "poly" or "polymer" used herein is used as a concept including "oligo" or "oligomer" which is a polymer in which a relatively small number of monomers are bonded.

In Formula (I), (II-a), or (II-b), for an alkyl moiety as defined for a variable group or moiety, in C₁-C₁₂ alkyl, C₁-C₂₁ alkylcarbonyl, C₁-C₄ alkylcarbonyl, C₁-C₂₁ alkylcarbonyl, or the like, for example, C₁-C₂₁ alkyl includes linear, branched, or cyclic alkyl having from 1 to 21 carbon atoms, and may be typically, but not limited to, methyl, ethyl, propyl, isopropyl, butyl, isobutyl, t-butyl, pentyl, isopentyl, hexyl, heptyl, octyl, isooctyl, 2-ethylhexyl, nonyl, isonononyl, decyl, isodecyl, undecyl, dodecyl, hexadecyl, cyclopentyl, cyclopentylmethyl, cyclohexyl, cyclohexylmethyl, cyclohexylmethyl, or the like.

Similarly, for example, the aryl moiety in arylcarbonyl or aryloxy has from 6 to 30 carbon atoms, and specific examples thereof include phenyl, tolyl, xylyl, ethylphenyl, naphthyl, and anthryl. The heteroaryl in heteroarylcarbonyl or heteroaryloxy may be, for example, pyridyl, pyrimidyl, furyl, tetrahydrofuryl, dioxolanyl, imidazolidyl, oxazolidyl, piperidyl, morpholinyl, or the like.

In particular, in the C₁-C₆ alkylcarbonyl included in the definition of R₁ in Formula (I), the short-chain fatty acid in PEG-b-poly (short-chain fatty acid vinyl) obtained according to the description of Patent Document 1 is preferably a group derived from, for example, acetic acid, propionic acid, butyric acid (or butanoic acid) isobutyric acid, or isovaleric acid.

In connection with the definition of R₁ or R₂ in Formula (I), (II-a), or (II-b), although it is described that 20% or more of m1, m2a or m2b is C(=O)CHCl₂, the 20% or more may be 40% or more or 60% or more in each formula. In connection with these definitions, each group other than C(=O)CHCl₂ may be present to the extent that it does not adversely affect the achievement of the subject matter disclosed herein or the objects of the invention.

With regard to the definition of L₁, L₂ₐ or L_{2b} in Formula (I), (II-a), or (II-b), when the entire structure of each copolymer differs depending on directionality in which each group described is bonded in each of these formulas, it is understood to be incorporated into each formula with the described directionality.

m1 is an integer of from 5 to 1000, and may be an integer of from 10 to 500, an integer of from 20 to 250, or an integer of from 50 to 160.

m2a or m2b is an integer of from 5 to 500, and may be an integer of from 10 to 500, an integer of from 20 to 250, or an integer of from 50 to 160.

On the other hand, n1 is an integer of from 5 to 500 and may be an integer of from 10 to 300, an integer of from 20 to 200, or an integer of from 50 to 160, and n2a or n2b is an integer of from 5 to 500 and may be an integer of from 10 to 300, an integer of from 20 to 200, or an integer of from 50 to 160.

The subject matter 4 defines a nano-sized polymeric micelle formed from the copolymer described in the subject matter 1 in an aqueous medium. As another aspect, the nano-sized polymeric micelle can be defined as follows, for example. A nano-sized polymeric micelle comprising the copolymer described in the subject matter 1 comprises:
a poly(ethylene glycol) segment in a shell of the polymeric micelle; and
a polymer segment carrying dichloroacetic acid in a side chain in a core (nucleus) of the polymeric micelle.

The terms "nano-sized polymeric micelle" and "nanoparticle" are used herein as interchangeably, wherein the term "nano-size" or "nano" means that when dynamic light scattering measurement (DLS) of polymeric micelles or nanoparticles in water is performed, an average diameter is in a nanometer order, and the average diameter is in a size of from about 10 nm to about 2000 nm, from about 10 nm to about 500 nm, or from about 25 nm to about 200 nm.

### <Production of copolymer represented by Formula (I)>

In a hydrophilic-hydrophobic copolymer comprising a block of a polymer segment having dichloroacetic acid in a hydrophobic segment side chain via an ester bond and poly(ethylene glycol) as a block of a hydrophilic segment, for example, the production of a copolymer represented by Formula (I) can be conveniently carried out by treating, under hydrolysis conditions known per se, a copolymer which is a part of a block copolymer represented by Formula (BC) described in Patent Document 1: 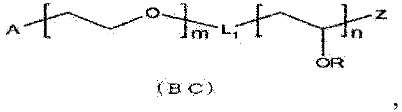 and, in particular, a copolymer in which R is -(C=O) R¹ and R¹ is, for example, alkyl having from 1 to 6 carbon atoms, thereby converting all or some of the OR groups into OH, and reacting dichloroacetic anhydride with the copolymer thus produced having OH to dichloroacetylate all or a part of the OH.

### <Production of copolymer represented by Formula (II-a) or (II-b)>

In a hydrophilic-hydrophobic copolymer comprising a block of a polymer segment having dichloroacetic acid in a hydrophobic segment side chain via an amide bond and poly(ethylene glycol) as a block of a hydrophilic segment, the production of a copolymer represented by Formula (II-a) can be conveniently carried out by reacting a copolymer obtained based on a method for synthesizing PEG-b-P (L-Orn) or PEG-b-P (L-Lys), which is a precursor in producing a copolymer containing a poly(L-arginine) segment or a poly(L-lysine-Gua) segment (where Gua means a guanidine group) described in WO2016/167333 A1, with dichloroacetic anhydride to dichloromethyloylate δ-position NH₂ of Orn or ε-position NH₂ of Lys.

Briefly, the production of PEG-b-P (L-Orn) or PEG-b-P (L-Lys) as a precursor can be carried out, for example, according to the following reaction scheme.

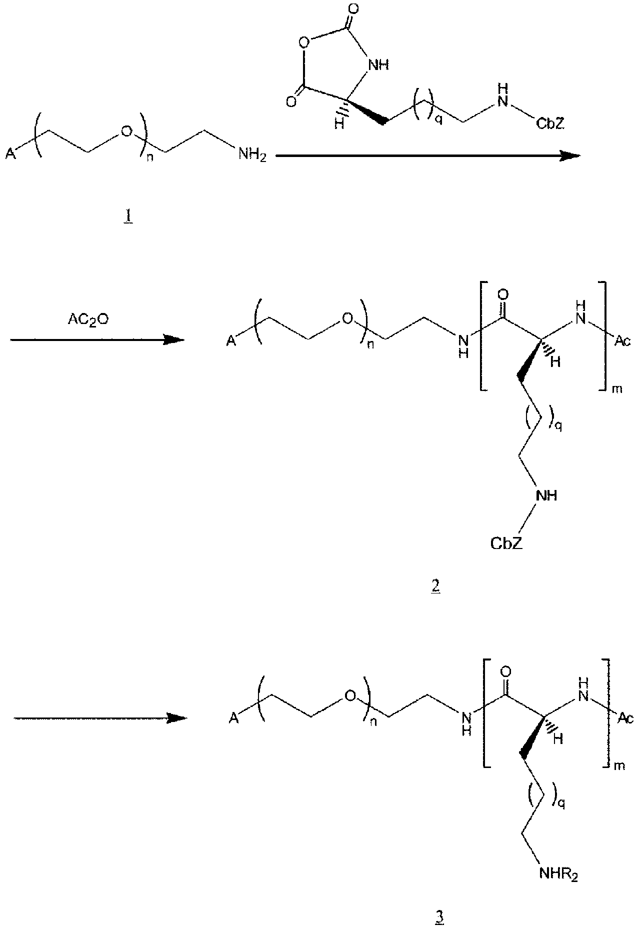

In the above formula, q is an integer of 1 or 2, Cbz represents benzyloxycarbonyl which is a protecting group of an amino group, and A, R₂, m, and n are each synonymous with A₂, R₂, m2a, and n2a as defined for Formula (II-a).

As another method, for example, a compound (some of these are commercially available) having oxy in which the α-terminal of PEG represented by 1 of the above reaction scheme is protected by a protecting group and having, at the ω-terminal oxy, -(CH₂)ₐ-NH₂ or -(CH₂)ₐ-OH corresponding to a linking group L₂ₐ in Formula (II-a) is provided, and on the other hand, a polymer (for example, the polymer can be prepared from those obtained by ring-opening polymerization of a corresponding amino acid N-carboxyanhydride (NCA) using an initiator which is easily removed) having m repeating unit represented by 2, in which the C-terminal is carboxyl and the N-terminal is represented by Za is provided. These are coupled in the presence of an appropriate dehydration-condensation agent, then the Cbz group is removed as necessary, and then the δ-position NH₂ of Orn or the ε-position NH₂ of Lys is dichloromethyloylated to perform production.

The copolymer represented by Formula (II-b) can be produced, for example, by providing a compound having oxy in which the α-terminal of PEG represented by 1 described above is protected by a protecting group and having, at the ω-terminal oxy, -(CH₂)_{b}-COOH corresponding to a linking group L_{2b} in Formula (II-b), on the other hand, providing a polymer having m repeating unit represented by 2, in which the N-terminal is a free amino group, and the C-terminal is represented by Zb, coupling these in the presence of an appropriate dehydration-condensation agent, then removing the Cbz group as necessary, and then dichloromethyloylating the δ-position NH₂ of Orn or the ε-position NH₂ of Lys.

### <Formation of polymeric micelle or nanoparticle>

The copolymer represented by Formula (I), (II-a), or (II-b) disclosed in the present specification is amphiphilic, if necessary, after preparing an aqueous solution containing a water-soluble organic solvent, for example, N,N-dimethylformamide (DMF) or dimethyl sulfoxide (DMSO), an unreacted raw material and the like are removed from a reaction mixture, and in order to associate and self-assemble a plurality of the copolymers, dialysis is performed against water via a dialysis membrane having a constant molecular weight cut-off, whereby a target nano-sized polymeric micelle or nanoparticle can be provided. The polymeric micelle or nanoparticle thus provided can be obtained as a separated solid by, for example, freeze-drying, centrifugation, and the like. When free NH₂ is present in forming a polymeric micelle from the copolymer represented by Formula (II-a) or (II-b), it is necessary to devise to stably form the polymeric micelle. For example, the pH of an aqueous solution containing the copolymer may be adjusted to be acidic, or as described in WO2016/167333 A1, although the present invention isnot limited thereto, a polyanionic polymer selected from the group consisting of a polyanionic polysaccharide, a polyanionic polypeptide, poly(acrylic acid), and poly(methacrylic acid) is allowed to co-exist, and formation of a polyion complex may be utilized.

The copolymer represented by Formula (II-a) or (II-b) preferably uses L-type Orn or Lys as a starting material, and may be produced using racemates of L- and D- types.

### <Pharmaceutical preparation>

The nanoparticles prepared as described above can prepare a tumor-specific radiosensitizer using the nanoparticles themselves as active ingredients. Since the nanoparticles can be dissolved or uniformly dispersed in an aqueous medium (aqueous solution capable of containing physiological saline and a pH adjusting agent as necessary) and can be reconstituted with water, the nanoparticles can be provided as formulations in various forms. Thus, a wide variety of preparations, for example, oral preparations in various forms including parenteral preparations (including agents for subcutaneous administration, intravenous infusion type injection agents, and the like) can be provided. In preparing a preparation or composition for oral preparations, either a normal pharmaceutical medium (for example, in the case of an oral liquid formulation such as water, glycol, oil, alcohol, perfume, preservative, and colorant, for example, a suspension, an elixir, and a solution) or a carrier (for example, in the case of an oral solid formulation such as starch, sugar, microcrystalline cellulose, diluent, granule, lubricant, binder, and disintegrant, for example, powders, hard and soft capsules and tablets) can be used. For example, when the oral dosage form is a tablet, a binder (for example, gum tragacanth, acacia, corn starch or gelatin), an excipient (for example, disodium phosphate), a disintegrant (for example, corn starch, potato starch, alginic acid), a lubricant (for example, sodium stearate), and a sweetener (for example, sucrose, lactose or sacchari can be included.

In such a pharmaceutical preparation, the optimal dose varies depending on a disease to be treated or an administration method, and thus the dose cannot be uniformly specified; however, the dose can be determined by a specialist based on data obtained through a small scale clinical trial and the like. For example, but not limited to, in terms of weight, at least 5 wt.% of the nanoparticles may be contained as an active ingredient. A proportion of the active ingredient in these formulations or compositions is variable and can conveniently be between about 30 wt.% and about 90 wt.% per total weight of the formulation or composition. Because the nanoparticles may be effective at a wide range of dosage, for example, doses of from about 0.1 to about 10,000 mg/day, preferably from about 1 to about 1000 mg/day, can be used in adult therapy. The optimal dose depends on a desired therapy, a form of administration, a subject to be treated, and a weight of the subject to be treated, an attending physician experience.

### <Pharmaceutical use>

As can be understood from each of Test Examples described later, a pharmaceutical preparation comprising, as an active ingredient, a nanoparticle formed from a copolymer represented by Formula (I), (II-a), or (II-b) can exhibit the above-described physiological activity of dichloroacetic acid itself at a local biological site of a tumor tissue to which the nanoparticle is delivered or a mammal used in the test. Therefore, the patient or individual referred to in the subject matter 9 is not limited to a human, and also includes mammals other than a human, in particular, pet animals, and for example, the tumor specific sensitizers and pharmaceutical preparations of the subject matter 5, the subject matter 6, and the like are not limited to a human, and also include mammals other than a human as a subject to be used.

In particular, according to Test Examples 1 and 2, it can be understood that the nanoparticles derived from the copolymer (including an ester bond) of Formula (I) exhibit more excellent effects than those derived from Formula: PEG-b-POrn (δ-position -NH) C(=O)Cl₂ synthesized in Production Example 2. Although this is not intended to limit the content of the present invention by theory, it is presumed that this is because a tumor in which inflow from a broken blood vessel (EPR effect) is more likely to occur was used in the test example in the former nanoparticles as compared to the latter nanoparticles. Although not bound by theory, in this test, it can be inferred that the nanoparticles derived from the latter formula reach tumor tissue before releasing the DCA, and the DCA is released therein by an enzyme from a cancer cell such as a matrix metalloprotease. As described above, the EPR effect may be weak depending on the type of tumor; however, in such a case, the time for stably staying in the blood stream is important, and the amide bond may exhibit a superior effect.

Therefore, according to the present invention, there is provided a pharmaceutical preparation for enhancing a therapeutic effect by being used in combination with radiation therapy of a tumor in addition to a tumor-specific sensitizer, the preparation including the copolymer described in the subject matter 1 or the polymeric micelle described in the subject matter 4 as an active ingredient, in other words, the preparation including nanoparticles derived from a copolymer represented by Formula (I) or a copolymer represented by Formula (II-a) or (II-b) as an active ingredient.

In addition, if the DCA has an action of converting an energy metabolism mode of cancer from a glycolytic system to a mitochondrial electron transport system as a PDK inhibitor, it can be inferred that the nanoparticles (capable of releasing DCA in vivo) can be widely used not only in combination with radiation therapy but also in other cancer therapies. In fact, it has been reported that the DCA in combination with cisplatin (see Cancer Lett. 2016; 371 (1): 20-29) or in combination with photodynamic therapy with 5-ALA (see PLoS One. 2018; 13 (10): e0206182) exhibits a certain effect, and in these combinations, it can be reasonably interpreted that it is also beneficial to use the nanoparticles derived from a copolymer represented by Formula (I), (II-a), or (II-b) instead of the DCA as an active ingredient in the above combination or photodynamic therapy.

### Description of Embodiments

Hereinafter, the subject matter or the present invention disclosed in this specification will be described more specifically with reference to specific examples, and the subject matter or the present invention disclosed in this specification is not limited thereto at all.

### Reference Production Example 1: Synthesis of poly(ethylene glycol)-b-poly(vinyl butyrate) (PEG-b-PVBu)

The title copolymer was produced in the same manner as described in "Example 5" in Patent Document 1. The resulting copolymer was referred to as N1193. The PEG used in this production method is of MW = 5000, and for the properties of the copolymer, refer to, for example, ¹H NMR spectrum and the like regarding the copolymer in Patent Document 1, if necessary.

The following Production Example 1 and Production Example 2 are each carried out according to the following simplified reaction scheme.

### <Simplified reaction scheme>

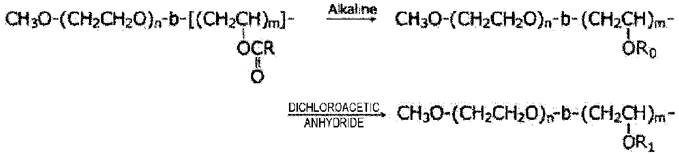

In the above formula, R₀ is C(=O)R or a hydrogen atom, at least one is a hydrogen atom, R₁ is a hydrogen atom, a C₁-C₆ alkylcarbonyl, or C(=O)CHCl₂, each R₁ may be different, and at least one is C(=O)CHCl₂. m and n correspond to m1 and n1, respectively, as defined for Formula (I).

Here, what is referred to as simplification is, for example, in consideration of omission of the ω-terminal group (S(=S)OCH₂CH₃) of the polymer main chain.

### Production Example 1: Synthesis of PEG-b-polyvinyl alcohol (PEG-b-PVA; N1223)

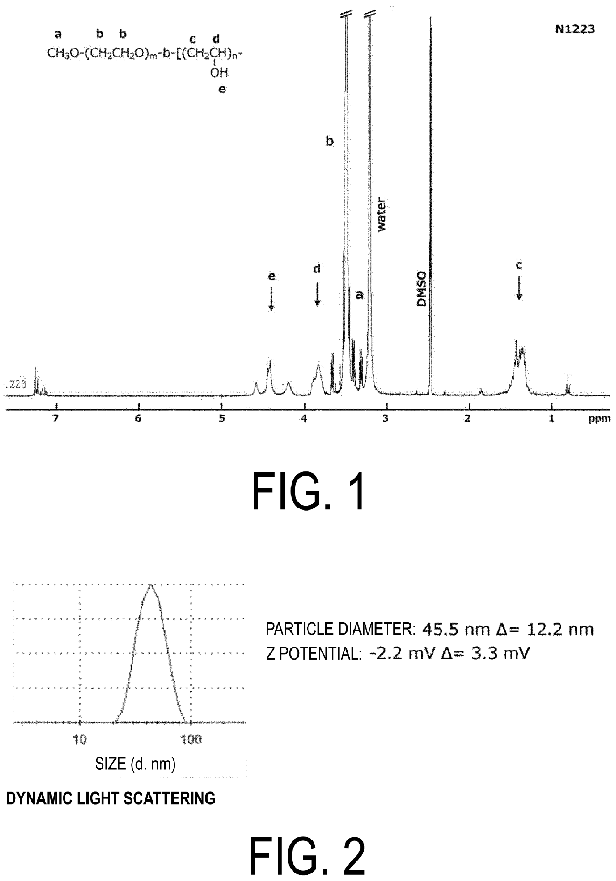

5 g of PEG-b-PVBu (N1193) was weighed in a 100 mL flask, 50 mL of methanol and 10 g of potassium carbonate (K₂CO₃) were added thereto, and the mixture was stirred at room temperature for 3 hours. After the reaction, the reaction solution was placed in a dialysis membrane tube (molecular weight cut-off MWCO = 3.5 kDa) and dialyzed against water to remove butyric acid, methanol, and calcium carbonate (1 day). The solution was concentrated to 60 mL and collected by lyophilization. A ¹H-NMR spectrum of the obtained PEG-b-PVA (N1223) is shown in FIG. 1.

### Production Example 2: Synthesis of PEG-b-poly(vinyl dichloroacetate) (PEG-b-PVCl₂Ac; N1228)

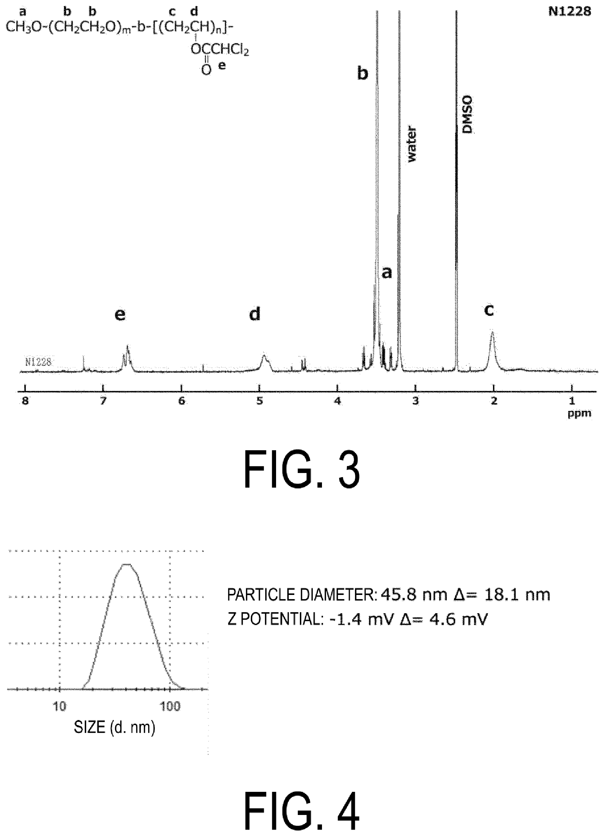

In a 100 mL flask, 2 g of PEG-b-PVA (N1223) and 50 mL of DMF were added and dissolved, then 5 mL of dichloroacetic anhydride was added and allowed to react at room temperature for 3 hours, and then the solution was placed in a dialysis membrane tube (MWCO = 3.5 kDa) and dialyzed against water to purify a target copolymer and form polymeric micelles from the copolymer. The solution was freeze-dried to recover the obtained polymeric micelle derived from the copolymer. FIG. 2 shows the measurement results of dynamic light scattering of the obtained polymeric micelle (N1228 derived nanoparticles, hereinafter sometimes abbreviated as EL-Nano^{DCA}), and FIG. 3 shows the ¹H-NMR spectrum of the recovered copolymer.

### Reference Production Example 2: Synthesis of poly(ethylene glycol)-b-poly(ornithine) (PEG-b-P (L-Orn))

The synthesis was carried out according to the method described in Example 1 and Example 2 of WO 2016/167333 A1 (N1182).

### Production Example 3: Synthesis of poly(ethylene glycol)-b-dichloromethyloylated poly(ornithine) (PEG-b-POrn (δ-position NH) -C(=O)CHCl₂: N1224)

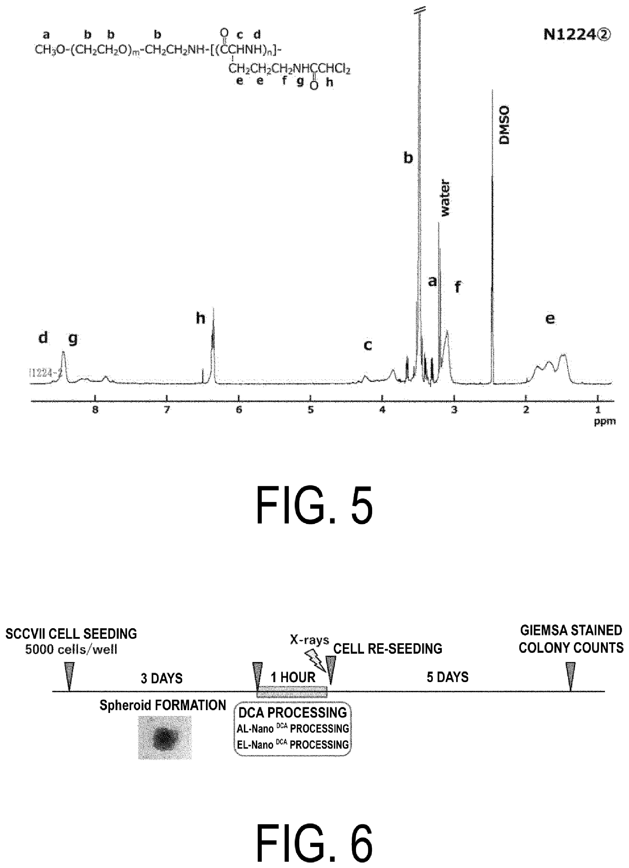

In a 100 mL flask, 2.5 g of PEG-b-P (L-Orn) (N1182) and 50 mL of DMF were added and dissolved, then 5 mL of dichloroacetic anhydride was added and reacted at room temperature for 1 hour, and then the reaction mixture was dialyzed (MWCO = 3.5 kDa) to obtain a purified solution. The solution was concentrated and freeze-dried to obtain a polymeric micelle derived from the copolymer. FIG. 4 shows the measurement results of dynamic light scattering of the obtained polymeric micelle (N1224 derived nanoparticles, hereinafter sometimes abbreviated as AL-Nano^{DCA}), and FIG. 5 shows the ¹H-NMR spectrum of the recovered target copolymer.

### Test Example 1: Confirmation of radiosensitizing effect

In this test, in order to verify the effect of the radiosensitizing effect of each of EL-Nano^{DCA} and AL-Nano^{DCA}, a colonization method using a spheroid was performed. As comparative subjects, a drug non-administration group and a dichloroacetic acid (DCA) administration group were also provided.

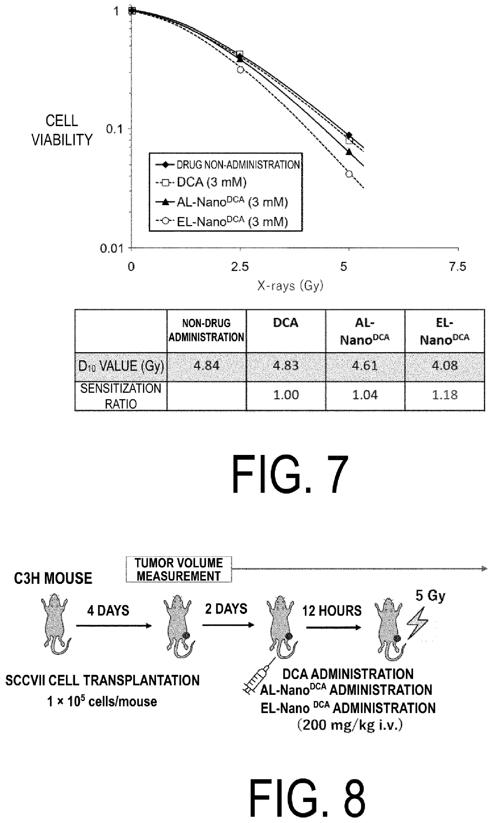

Mouse squamous cell cancer-derived SCCVII cells were seeded at 5000 cells/well in a U-shaped 96 well plate, and cultured for 3 days at 4°C and 5%CO₂ to produce a spheroid. DCA, EL-Nano^{DCA}, and AL-Nano^{DCA} were each added at 3 mM, and X-ray irradiation was performed. After the spheroids were dispersed using Accumax, an appropriate number of spheroids were seeded in a 60 mm plastic petri dish and cultured for 5 days. The cells were fixed for 10 minutes using methanol and stained with 2% Giemsa stain. Colonies containing 50 or more cells were counted, and a cell viability was calculated by normalization with a colony formation rate of a non-irradiated control group. An overview of an experimental schedule for the colonization method is shown in FIG. 6. A change in cell viability with respect to an irradiation dose is shown in FIG. 7. The cell viability was in the order of the drug non-administration group > DCA > AL-Nano^{DCA} > EL-Nano^{DCA}, a radiosensitization ratio to the drug non-administration group was 1.00 for DCA, 1.04 for AL-Nano^{DCA}, and 1.18 for EL-Nano^{DCA}, and only EL-Nano^{DCA} showed sensitization. The sensitization ratio was calculated based on the dose (D₁₀ value) that gives a cell viability of 10%.

### Test Example 2: Radiosensitizing effect in vivo

(a) An animal experiment using a mouse (C3H/HeJYOk Slc, male, 6 weeks old) was performed. 1.0 × 10⁵ SCCVII cells were transplanted into right thigh of a mouse, and animal experiments were performed according to the schedule shown in FIG. 8 for the following 8 groups (n = 5) randomly divided. During the experiment, tumor volume measurements were performed daily.
   (1) Drug non-administration group
   (2) DCA (200 mg/kg) administration group
   (3) AL-Nano^{DCA} (200 mg/kg) administration group
   (4) EL-Nano^{DCA} (200 mg/kg) administration group
   (5) Drug non-administration + 5 Gy group
   (6) DCA (200 mg/kg) administration + 5 Gy group
   (7) AL-Nano^{DCA} (200 mg/kg) administration + 5 Gy group
   (8) EL-Nano^{DCA} (200 mg/kg) administration + 5 Gy group

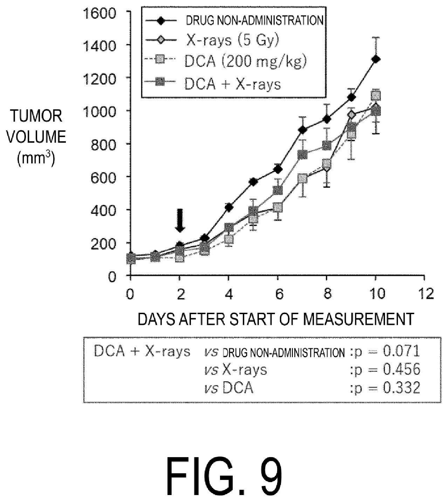

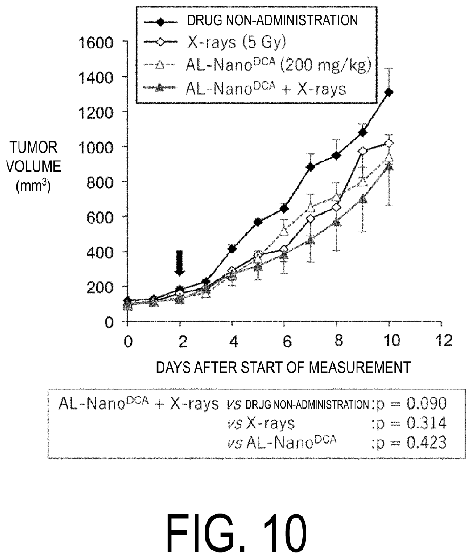

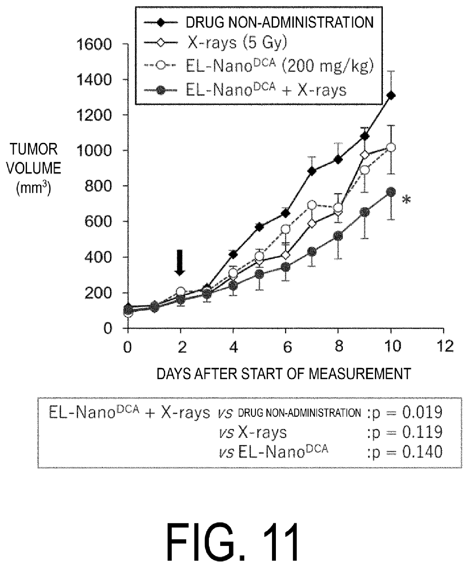

A change in tumor volume before and after each treatment is shown in FIG. 9 for the result associated with the use of DCA alone or in combination with X-ray, in FIG. 10 for the result associated with the use of AL-Nano^{DCA} alone or in combination with X-ray, and in FIG. 11 for the result associated with the use of EL-Nano^{DCA} alone or in combination with X-ray, respectively. The arrows described in these figures indicate treatment days, and a p value after a statistical significance test at day 10 from the start of the experiment is shown in a lower part of each graph. From these figures, regarding DCA and AL-Nano^{DCA}, no significant antitumor effect was observed even in the treatment with the drug alone or in combination with X-ray. On the other hand, regarding EL-Nano^{DCA}, a significant antitumor effect was observed by treatment in combination with X-ray, and a tendency to exhibit the sensitizing effect was also observed for each single treatment.

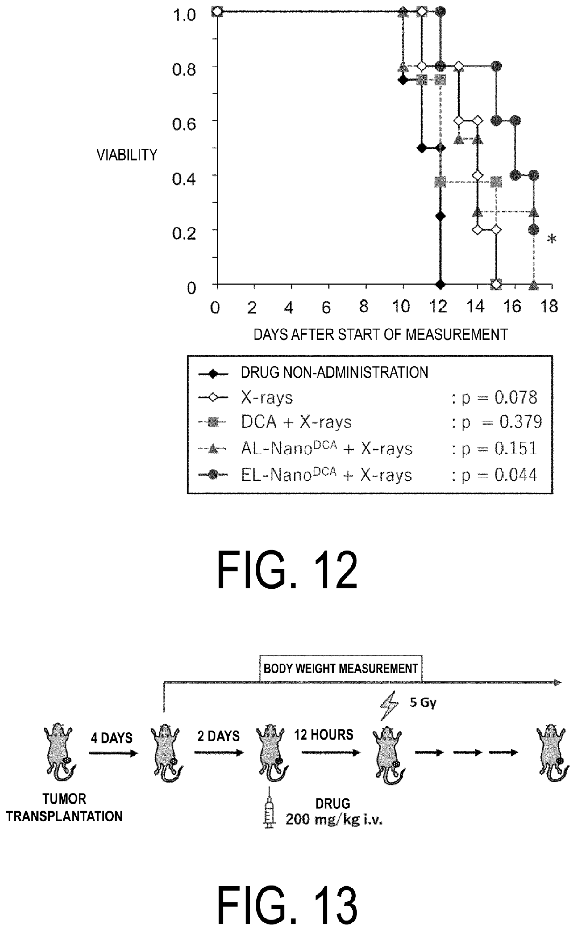

In addition, a viability curve of tumor-bearing mice at the time of combined treatment with X-ray for DCA, AL-Nano^{DCA}, or EL-Nano^{DCA} is shown in FIG. 12. From the viability curve using this tumor endpoint (the time point at which the tumor volume in FIGS. 9 to 11 exceeds 1500 mm³) as an index, significant prolongation of survival time was observed only with EL-Nano^{DCA} in combination with X-ray. Here, the tumor endpoint is not death of a mouse, and a point at which the tumor volume exceeds 1500 mm³ is determined as death as a humane endpoint.

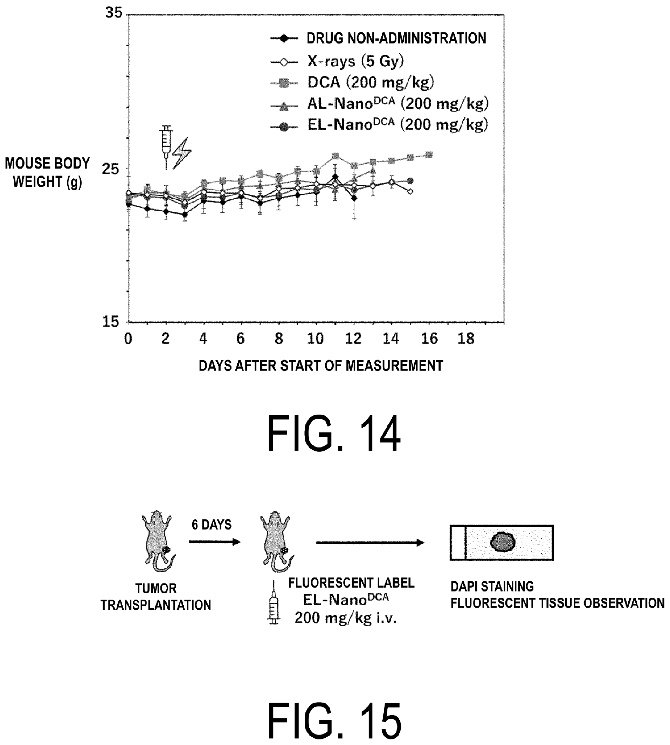

(b) In the above (a), a temporal change of a mouse body weight as well as the tumor volume was also recorded (for reference, see FIG. 13 showing a schedule of an animal experiment). In addition to the single X-ray irradiation, the results of recording a body weight change by the treatment with the drug alone are shown in FIG. 14. No significant body weight loss was observed by the administration of DCA, AL-Nano^{DCA}, and EL-Nano^{DCA} with respect to the body weight change of the drug non-administration group, and the safety of the drug could be confirmed at the dose of 200 mg/kg.

### Test Example 3: Tumor tissue distribution after administration of nanoparticulated DCA in vivo

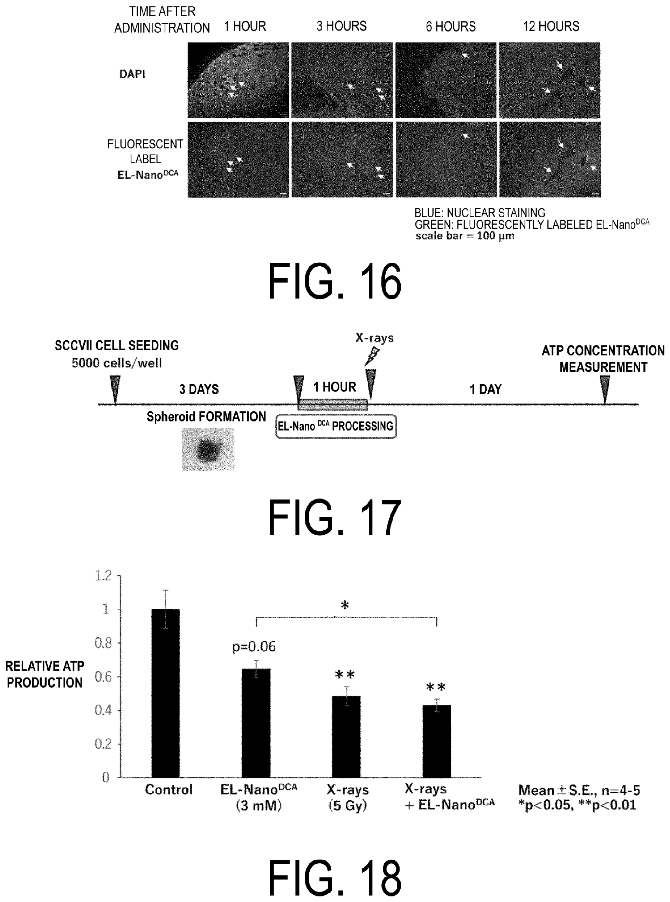

Using TAMRA-EL-Nano^{DCA} labeled with a fluorescent dye TAMRA (carboxy tetramethylrhodamine), whether EL-Nano^{DCA} was accumulated in the tumor was evaluated. TAMRA-EL-Nano^{DCA} (200 mg/kg) was administered from a tail vein of a SCCVII implanted tumor mouse, and then tumor tissues were then collected at any time (see FIG. 15 for an overview of the schedule of the animal experiment). A cell nucleus of a tissue section was stained with a fluorescent dye DAPI and observed with a fluorescence microscope. The observation image is shown in FIG. 16. An image in which TAMRA-EL-Nano^{DCA} is accumulated around the blood vessel indicated by the white arrow is observed in any of 1 hour, 3 hours, 6 hours, and 12 hours after administration of TAMRA-EL-Nano^{DCA}.

### Test Example 4: Influence of nanoparticulated DCA and X-ray treatment on energy production

In order to examine a mechanism by which EL-Nano^{DCA} exhibits a radiosensitizing effect, an influence on energy metabolism of cells was examined using an amount of ATP production as an index. SCCVII cells cultured in a three-dimensional spheroid were treated with 3 mM of EL-Nano^{DCA} for 1 hour before X-ray irradiation, and an intracellular ATP amount after 24 hours was measured. (See FIG. 17 for an overview of a schedule of experiment using cells). The measurement results are shown in FIG. 18. From FIG. 18, the intracellular ATP amount showed a decreasing tendency by EL-Nano^{DCA} treatment, and a significant decrease by X-ray treatment and a combined treatment of EL-Nano^{DCA} and X-ray. In particular, in the combined use group, the intracellular ATP amount is significantly reduced as compared with EL-Nano^{DCA} alone, suggesting a strong inhibitory effect on energy metabolism by the combined use.

## Claims

1. A hydrophilic-hydrophobic copolymer comprising:
a block comprising a poly(ethylene glycol) segment; and
a block comprising a polymer segment carrying dichloroacetic acid in a side chain,
wherein the hydrophilic-hydrophobic copolymer is represented by Formula (I), (II-a), or (II-b): 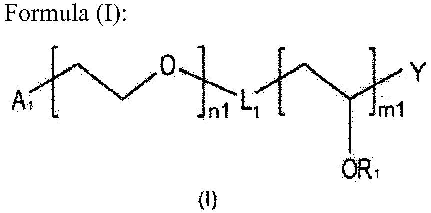
where
A₁ represents
(i) unsubstituted or substituted C₁-C₁₂ alkyloxy, a substituent in a case of being substituted is a formyl or a group of Formula R^{a}R^{b}CH-, (where R^{a} and R^{b} are independently C₁-C₄ alkyloxy or R^{a} and R^{b} together represent -OCH₂CH₂O-, -O(CH₂)₃O-, or -O(CH₂)₄O-), or
(ii) a group represented by the following formula: 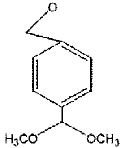
L₁ is selected from groups represented by 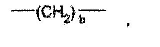 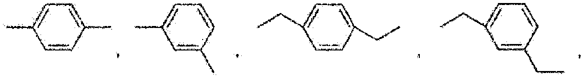
or the group consisting of (CH₂)_{c}S, CO(CH₂)_{c}S, (CH₂)_{c}NH, (CH₂)_{c}CO, CO, OCOO, and CONH, with the proviso that b is an integer of from 2 to 6, and c is each independently an integer of from 1 to 5,
R₁ is a hydrogen atom, a C₁-C₆ alkylcarbonyl, or C(=O)CHCl₂, and each R₁ is optionally different, with the proviso that 20% or more of m1 is C(=O)CHCl₂,
Y is a hydrogen atom, SH, S (C=S)-Ph, S(=S)OCH₂CH₃, a hydroxyl group, a C₁-C₆ alkyloxy group, or an aryl-C₁-C₂ alkyloxy group,
m1 is an integer of from 5 to 1000, and
n1 is an integer of from 5 to 500; 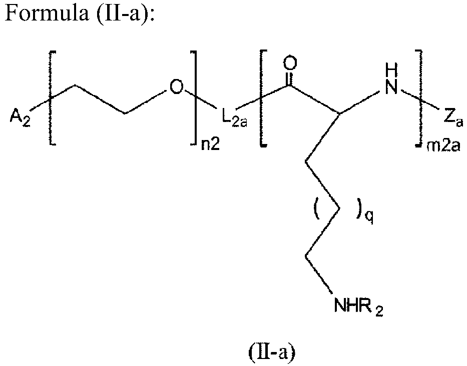
where
A₂ has the same meaning as defined for A₁ of Formula (I)
L₂ₐ represents (CH₂)ₐ-NH or (CH₂)ₐ-O, with the proviso that a is each independently an integer of from 1 to 6,
R₂ is a hydrogen atom, benzyloxycarbonyl (Cbz), t-butoxycarbonyl, C₁-C₆ alkylcarbonyl, or C(=O)CHCl₂, and each R₂ is optionally different, with the proviso that 20% or more of m2a is C(=O)CHCl₂,
Za represents H, C₁-C₂₁ alkylcarbonyl, substituted C₁-C₄ alkylcarbonyl, unsubstituted or substituted C₃-C₇ cycloalkylcarbonyl, unsubstituted or substituted arylcarbonyl, or unsubstituted or substituted 5 or 6-membered heteroarylcarbonyl, where a substituent in a case of being substituted is optionally one or more C₁-C₄ alkyloxy, phenyl, and a fluorine atom,
m2a is an integer of from 5 to 500,
n2a is an integer of from 5 to 500, and
q is an integer of 1 or 2; 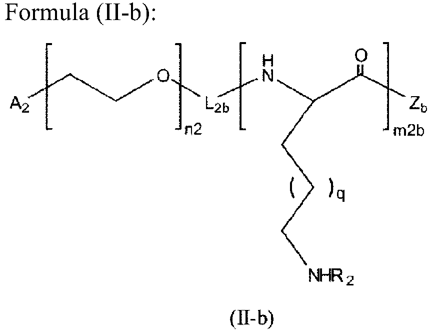
where
A₂ has the same meaning as defined for A₁ of Formula (I)
L_{2b} represents (CH₂)_{b}-C(=O), with the proviso that _{b} is an integer of from 1 to 6,
R₂ is a hydrogen atom, benzyloxycarbonyl (Cbz), t-butoxycarbonyl, C₁-C₆ alkylcarbonyl or C(=O)CHCl₂, and each R₂ is optionally different, with the proviso that 20% or more of m2b is C(=O)CHCl₂,
Zb represents OH, unsubstituted or substituted C₁-C₂₁ alkyloxy, unsubstituted or substituted C₃-C₇ cycloalkyloxy, unsubstituted or substituted aryloxy, or unsubstituted or substituted 5 or 6-membered heteroaryloxy, where a substituent in a case of being substituted is optionally one or more C₁-C₄ alkyloxy, phenyl, and a fluorine atom,
m2b is an integer of from 5 to 500,
n2b is an integer of from 5 to 500, and
q is an integer of 1 or 2.

2. The hydrophilic-hydrophobic copolymer according to claim 1, wherein the hydrophilic-hydrophobic copolymer is represented by Formula (I).

3. The hydrophilic-hydrophobic copolymer according to claim 1, wherein the hydrophilic-hydrophobic copolymer is represented by Formula (II-a) or (II-b).

4. A nano-sized polymeric micelle formed from the hydrophilic-hydrophobic copolymer described in claim 1 in an aqueous medium.

5. A tumor-specific radiosensitizer comprising, as an active ingredient, the hydrophilic-hydrophobic copolymer described in claim 1 or a nano-sized polymeric micelle formed from the hydrophilic-hydrophobic copolymer in an aqueous medium.

6. A pharmaceutical preparation for enhancing a therapeutic effect by being used in combination with radiation therapy of a tumor, the pharmaceutical preparation comprising, as an active ingredient, the hydrophilic-hydrophobic copolymer described in claim 1 or a nano-sized polymeric micelle formed from the hydrophilic-hydrophobic copolymer in an aqueous medium.

7. The hydrophilic-hydrophobic copolymer according to claim 1 or the nano-sized polymeric micelle according to claim 4 for use as a tumor-specific radiosensitizer.

8. The hydrophilic-hydrophobic copolymer according to claim 1 or the nano-sized polymeric micelle according to claim 4 for use in a pharmaceutical preparation for enhancing a therapeutic effect by being used in combination with radiation therapy of a tumor.

9. A method for enhancing a therapeutic effect on a tumor, the method comprising administering an effective amount of the hydrophilic-hydrophobic copolymer described in claim 1 or a nano-sized polymeric micelle formed from the hydrophilic-hydrophobic copolymer in an aqueous medium to a patient or an individual who is scheduled to receive or is actually receiving radiation therapy for a tumor.
